# EUROPEAN PATENT SPECIFICATION

(11) **EP 2 021 799 B1**
(45) Date of publication and mention of the grant of the patent: **20.07.2011**
(21) Application number: 07811859.3
(22) Date of filing: 27.04.2007
(51) Int. Cl.: G01N 33/53, C07K 14/00, C07K 16/00, G01N 33/68

(54) **INTERLEUKIN-33 (IL-33) FOR THE DIAGNOSIS AND PROGNOSIS OF CARDIOVASCULAR DISEASE**
INTERLEUKIN-33 (IL-33) ZUR DIAGNOSE UND VORHERSAGE VON HERZ-GEFÄSS-ERKRANKUNGEN
INTERLEUKINE-33 (IL-33) UTILISÉE POUR LE DIAGNOSTIC ET LE PRONOSTIC DES MALADIES CARDIOVASCULAIRES

(30) Priority: 27.04.2006 US 795473 P
(43) Date of publication of application: 11.02.2009
(73) Proprietor: Critical Care Diagnostics, Inc., New York, New York 10019 (US)
(72) Inventor: SNIDER, James, Pleasanton, CA 94588 (US); JACOBSON, Sven, New York, New York 10001 (US)
(74) Representative: Fleck, Barbara
(86) International application number: PCT/US2007/067626
(87) International publication number: WO 2007/143295

(56) References cited:
- WO-A-2005/079844
- WO-A-2007/127749
- WO-A-2007/130627
- WO-A-2007/131031
- US-A1- 2008 003 199
- SHIMPO MASAHISA ET AL: "Serum levels of the interleukin-1 receptor family member ST2 predict mortality and clinical outcome in acute myocardial infarction" CIRCULATION,, vol. 109, no. 18, 11 May 2004 (2004-05-11), pages 2186-2190, XP002523097
- SCHMITZ J ET AL: "IL-33, an interleukin-1-like cytokine that signals via the IL-1 receptor-related protein ST2 and induces T helper type 2-associated cytokines" IMMUNITY, CELL PRESS, US, vol. 23, no. 5, 1 November 2005 (2005-11-01), pages 479-490, XP002432892 ISSN: 1074-7613
- KAKKAR R ET AL: "The IL-33/ST2 pathway: Therapeutic target and novel biomarker" NATURE REVIEWS DRUG DISCOVERY 2008 GB, vol. 7, no. 10, 2008, pages 827-840, XP002530680 ISSN: 1474-1776 1474-1784

### SEQUENCE LISTING

<110> Critical Care Dagnostics, Inc.
<120> INTERLEUKIN-33 (IL- 33) FOR THE DIAGNOSIS AND PROGNOSIS OF CARDIOVASCULAR DISEASE
<130> 20060- 004WO1
<140> PCT/ US2007/ 067626 <141>2007- 04- 27
<150> US 60/795,473 <151> 2006-04-27
<160> 2
<170> Fast SEQ for Windows Version 4.0
<210> 1
   <211> 2644
   <212> DNA
   <213> Homo sapiens
<400> 1
   caacagaata ctgaaaaatg aagcctaaaa tgaagtattc aaccaacaaa atttccacag 60
   caaagtggaa gaacacagca agcaaagcct tgtgtttcaa gctgggaaaa tcccaacaga 120
   aggccaaaga agtttgcccc atgtacttta tgaagctccg ctctggcctt atgataaaaa 180
   aggaggcctg ttactttagg agagaaacca ccaaaaggcc ttcactgaaa acaggtagaa 240
   agcacaaaag acatctggta ctcgctgcct gtcaacagca gtctactgtg gagtgctttg 300
   cctttggtat atcaggggtc cagaaatata ctagagcact tcatgattca agtatcacag 360
   gaatttcacc tattacagag tatcttgctt ctctaagcac atacaatgat caatccatta 420
   cttttgcttt ggaggatgaa agttatgaga tatatgttga agacttgaaa aaagatgaaa 480
   agaaagataa ggtgttactg agttactatg agtctcaaca cccctcaaat gaatcaggtg 540
   acggtgttga tggtaagatg ttaatggtaa ccctgagtcc tacaaaagac ttctggttgc 600
   atgccaacaa caaggaacac tctgtggagc tccataagtg tgaaaaacca ctgccagacc 660
   aggccttctt tgtccttcat aatatgcact ccaactgtgt ttcatttgaa tgcaagactg 720
   atcctggagt gtttataggt gtaaaggata atcatcttgc tctgattaaa gtagactctt 780
   ctgagaattt gtgtactgaa aatatcttgt ttaagctctc tgaaacttag ttgatggaaa 840
   cctgtgagtc ttgggttgag tacccaaatg ctaccactgg agaaggaatg agagataaag 900
   aaagagacag gtgacatcta agggaaatga agagtgctta gcatgtgtgg aatgttttcc 960
   atattatgta taaaaatatt ttttctaatc ctccagttat tcttttattt ccctctgtat 1020
   aactgcatct tcaatacaag tatcagtata ttaaataggg tattggtaaa gaaacggtca 1080
   acattctaaa gagatacagt ctgaccttta cttttctcta gtttcagtcc agaaagaact 1140
   tcatatttag agctaaggcc actgaggaaa gagccatagc ttaagtctct atgtagacag 1200
   ggatccattt taaagagcta cttagagaaa taatttccca cagttccaaa cgataggctc 1260
   aaacactaga gctgctagta aaaagaagac cagatgcttc acagaattat catttttcca 1320
   actggaataa aacaccaggt ttgtttgtag atgtcttagg caacactcag agcagatctc 1380
   ccttactgtc aggggatatg gaacttcaaa ggcccacatg gcaagccagg taacataaat 1440
   gtgtgaaaaa gtaaagataa ctaaaaaatt tagaaaaata aatccagtat ttgtaaagtg 1500
   aataacttca tttctaattg tttaattttt aaaattctga tttttatata ttgagtttaa 1560
   gcaaggcatt cttacacgag gaagtgaagt aaattttagt tcagacataa aatttcactt 1620
   attaggaata tgtaacatgc taaaactttt ttttttttaa agagtactga gtcacaacat 1680
   gttttagagc atccaagtac catataatcc aactatcatg gtaaggccag aaatcttcta 1740
   acctaccaga gcctagatga gacaccgaat taacattaaa atttcagtaa ctgactgtcc 1800
   ctcatgtcca tggcctacca tcccttctga ccctggcttc cagggaccta tgtcttttaa 1860
   tactcactgt cacattgggc aaagttgctt ctaatcctta tttcccatgt gcacaagtct 1920
   ttttgtattc cagcttcctg ataacactgc ttactgtgga atattcattt gacatctgtc 1980
   tcttttcatt tcttttaact accatgccct tgatatatct tttgcacctg ctgaacttca 2040
   tttctgtatc acctgacctc tggatgccaa aacgtttatt ctgctttgtc tgttgtagaa 2100
   ttttagataa agctattaat ggcaatattt ttttgctaaa cgtttttgtt ttttactgtc 2160
   actagggcaa taaaatttat actcaaccat ataataacat tttttaacta ctaaaggagt 2220
   agtttttatt ttaaagtctt agcaatttct attacaactt ttcttagact taacacttat 2280
   gataaatgac taacatagta acagaatctt tatgaaatat gaccttttct gaaaatacat 2340
   acttttacat ttctacttta ttgagaccta ttagatgtaa gtgctagtag aatataagat 2400
   aaaagaggct gagaattacc atacaagggt attacaactg taaaacaatt tatctttgtt 2460
   tcattgttct gtcaataatt gttaccaaag agataaaaat aaaagcagaa tgtatatcat 2520
   cccatctgaa aaacactaat tattgacatg tgcatctgta caataaactt aaaatgatta 2580
   ttaaataatc aaatatatct actacattgt ttatattatt gaataaagta tattttccaa 2640
   atgt 2644
<210> 2
   <211> 270
   <212> PRT
   <213> Homo sapiens
<400> 2
   Met Lys Pro Lys Met Lys Tyr Ser Thr Asn Lys Ile Ser Thr Ala Lys
   1               5                   10                  15
   Trp Lys Asn Thr Ala Ser Lys Ala Leu Cys Phe Lys Leu Gly Lys Ser
               20                  25                  30
   Gln Gln Lys Ala Lys Glu Val Cys Pro Met Tyr Phe Met Lys Leu Arg
           35                  40                  45
   Ser Gly Leu Met Ile Lys Lys Glu Ala Cys Tyr Phe Arg Arg Glu Thr
       50                  55                  60
   Thr Lys Arg Pro Ser Leu Lys Thr Gly Arg Lys His Lys Arg His Leu
   65                  70                  75                  80
   Val Leu Ala Ala Cys Gln Gln Gln Ser Thr Val Glu Cys Phe Ala Phe
                   85                  90                  95
   Gly Ile Ser Gly Val Gln Lys Tyr Thr Arg Ala Leu His Asp Ser Ser
               100                 105                 110
   Ile Thr Gly Ile Ser Pro Ile Thr Glu Tyr Leu Ala Ser Leu Ser Thr
           115                 120                 125
   Tyr Asn Asp Gln Ser Ile Thr Phe Ala Leu Glu Asp Glu Ser Tyr Glu
       130                 135                 140
   Ile Tyr Val Glu Asp Leu Lys Lys Asp Glu Lys Lys Asp Lys Val Leu
   145                 150                 155                 160
   Leu Ser Tyr Tyr Glu Ser Gln His Pro Ser Asn Glu Ser Gly Asp Gly
                   165                 170                 175
   Val Asp Gly Lys Met Leu Met Val Thr Leu Ser Pro Thr Lys Asp Phe
               180                 185                 190
   Trp Leu His Ala Asn Asn Lys Glu His Ser Val Glu Leu His Lys Cys
           195                 200                 205
   Glu Lys Pro Leu Pro Asp Gln Ala Phe Phe Val Leu His Asn Met His
       210                 215                 220
   Ser Asn Cys Val Ser Phe Glu Cys Lys Thr Asp Pro Gly Val Phe Ile
   225                 230                 235                 240
   Gly Val Lys Asp Asn His Leu Ala Leu Ile Lys Val Asp Ser Ser Glu
                   245                 250                 255
   Asn Leu Cys Thr Glu Asn Ile Leu Phe Lys Leu Ser Glu Thr
               260                 265                 270

## Description

### TECHNICAL FIELD

This invention pertains to *in vitro* methods and compositions for the diagnosis and prognosis of cardiovascular conditions.

### BACKGROUND

Despite significant advances in therapy, cardiovascular disease remains the single most common cause of morbidity and mortality in the developed world. Thus, prevention and therapy of cardiovascular conditions such as heart failure, myocardial infarction and stroke is an area of major public health importance. Currently, several risk factors for future cardiovascular disorders have been described and are in wide clinical use in the detection of subjects at high risk. Such screening tests include evaluations of total and HDL cholesterol levels. However, a large number of cardiovascular disorders occur in subjects with apparently low to moderate risk profiles, and ability to identify such patients is limited. Moreover, accumulating data suggests that the beneficial effects of certain preventative and therapeutic treatments for patients at risk for or known to have cardiovascular disorders differs in magnitude among different patient groups.

Shimpo et al ("Serum levels of interleukin-1 receptor family member ST2 predict mortality in clinical outcome in acute myocardial infarction", Circulation, Vol. 109, No. 18, 11 May 2004, pages 2186-2190) relates to a study designed to test the hypothesis that ST2 serum levels are associated with risk of death and heart failure in patients presenting with ST elevation myocardial infarction, but is not concerned with diagnosis. Schmitz et al ("IL-33, an interleukin-1-like cytokine that signals via the IL-1 receptor-related protein ST2 and induces T helper type 2-associated cytokines", Immunity, Vol. 23, No. 5, 1 November 2005, pages 479-490) reports the induction of IL-33 expression in primary lung or dermal fibroblasts and keratinocytes upon TNF-α and IL-1-β activation. Induction in such cell types correlates with higher endogenous levels of IL-33 in lung and skin.

### SUMMARY

The present invention includes *in virto* methods for the use of interleukin-33 (IL-33) in the diagnosis and prognosis of cardiovascular conditions including acute coronary syndrome (ACS), coronary artery disease (CAD), myocardial infarction, heart failure, angina, cardiac hypertrophy, arterioselerosis, myocarditis, pericarditis, endocarditis, stroke and/or pulmonary embolism.

In one aspect, the invention provides in vitro methods for diagnosing a cardiac disease in a subject. The methods include determining a level of IL-33 in a sample from a subject, wherein the level of IL-33 in the sample is indicative of whether the subject has a cardiovascular disease (i.e., correlates to the presence or absence of cardiovascular disease). The methods include determining a level of IL-33 in the sample; and comparing the level of IL-33 in the sample to a reference level of IL-33. The level of IL-33 in the sample as compared to the reference indicates whether the subject has a cardiovascular disease.

In another aspect, the invention provides in vitro methods for determining the severity of a cardiovascular disease in a subject. The methods include determining a level of IL-33 in a sample from a subject, wherein the level of IL-33 in the sample is indicative of (correlates to) the severity of the cardiovascular disease in the subject. The methods include determining a level of IL-33 in the sample; and comparing the level of IL-33 in the sample to a reference level of IL-33. The level of IL-33 in the sample as compared to the reference indicates the severity of the cardiovascular disease in the subject.

In some embodiments of the methods described herein, the sample comprises blood, serum or plasma. In some embodiments, the sample comprises serum.

The reference level represents a level in a subject who does not have cardiovascular disease. In some methods, the reference level represents a level in a subject with a preselected severity of cardiovascular disease.

In some embodiments of the methods described herein, determining a level of IL-33 in the sample includes determining a level of one, two, or all three of mature IL-33, pre-IL-33, and pro-IL-33. Determining a level of IL-33 in the sample can include contacting a binding composition to the sample, wherein the binding composition specifically binds to IL-33, and measuring or determining the specific binding of the binding composition to the sample. Suitable binding compositions include antibodies that bind specifically to IL-33 polypeptide, and oligonucleotide probes that bind specifically to IL-33 polynucleotide.

In some embodiments, the cardiac disease diagnosed by a method described herein is acute coronary syndrome (ACS), myocardial infarction, heart failure, angina, cardiac hypertrophy, arteriosclerosis, myocarditis, pericarditis, endocarditis, stroke, and/or pulmonary embolism.

In some embodiments, the methods described herein also include determining a level in the sample of one or more other biomarkers, e.g., biomarkers selected from the group consisting of ST2, NT-proBNP, BNP, NT-proANP, and ANP, troponin, IMA, IL-6, CRP, creatinine, D-dimers, BUN, liver function enzymes, albumin, and bacterial endotoxin.

We also describe herein kits for diagnosing cardiovascular disease. The kits include an antibody that specifically binds to IL-33, and/or an oligonucleotide probe that specifically binds to a nucleic acid encoding IL-33, and instructions for use in a method described herein.

"Upregulated," as used herein, refers to increased expression of a gene and/or its encoded polypeptide. "Increased expression" refers to increasing (i.e., to a detectable extend) replication, transcription, and/or translation of IL-33, since upregulation of any of these processes results in an increase in concentration/amount of the polypeptide encoded by the gene. Conversely, "downregulation," or "decreased expression" as used herein, refers to reduced replication, transcription, and/or translation of the IL-33 gene and/or its encoded polypeptide. The upregulation or downregulation of gene expression can be directly determined by detecting an increase or decrease, respectively, in the level of mRNA for the gene, or the level of protein expression of the gene-encoded polypeptide, using any suitable means known to the art, such as nucleic acid hybridization or antibody detection methods, respectively, and in comparison to controls. "Expression," as used herein, refers to nucleic acid and/or polypeptide expression.

A "cardiac cell," as used herein, refers to a cardiomyocyte.

As used herein, a "subject" is a mammal or a non-human mammal. In general, human nucleic acids, polypeptides, and human subjects are preferred for use in diagnosing human patients.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting.

Other features and advantages of the invention will be apparent from the following detailed description and figures, and from the claims.

### BRIEF DESCRIPTION OF THE DRAWINGS

FIG. 1 is a representation of the nucleic acid sequence of human IL-33 (SEQ ID NO:1).
FIG. 2 is a representation of the amino acid sequence of human IL-33 (SEQ ID NO:2).

### DETAILED DESCRIPTION

It has previously been described that levels of the interleukin-1 receptor-like 1 (IL1RL1) protein can be used to diagnose cardiovascular disease and determine the prognosis for a patient with cardiovascular disease. The ligand for IL1RL1 has been described, and named IL-33 (see, e.g., Schmitz et al., Immunity 23(5):479-90 (2005); U.S. Pat. Pub. No. 2005/0203046). The present methods include the measurement of levels of IL-33 for the diagnosis and prognosis of cardiovascular disease.

### General Methodology

In general, the methods described herein include evaluating levels of IL-33 in a biological sample (e.g., a blood, serum, plasma, urine, or body tissue sample) from a subject, e.g., a mammal, e.g., a human. These levels provide diagnostic information indicating whether the subject has a cardiac disease, as described herein. In some embodiments, the level of IL-33 is determined once, e.g., at presentation. In some embodiment, the level of IL-33 is determined at any one or more of 1, 2, 3, 4, 5, 6, 7, 8, 12, 18, and/or 24 hours, and/or at 1-7 days after the onset of symptoms.

In embodiments where the level of IL-33 is determined more than once, the higher level can be used, or the change in levels can be determined and used. Levels of IL-33 can be determined multiple times to evaluate a subject's response to a treatment. For example, a level of IL-33 taken after administration of a treatment, e.g., one or more doses or rounds of a treatment, can be compared to levels of IL-33 before the treatment was initiated, e.g., a baseline level. The change in IL-33 levels would indicate whether the treatment was effective; e.g., a reduction in IL-33 levels would indicate that the treatment was effective.

In some embodiments, the level of IL-33 can be measured in patients previously diagnosed with a cardiovascular condition who are undergoing treatment on an outpatient basis, to monitor the condition of the patient and determine whether their condition is improving, stable or deteriorating. Levels of IL-33 can be measured at one or more times and compared to previous measurements, where an increase in IL-33 relative to an earlier measurement would indicate that the patient's condition is deteriorating, suggesting that the current treatment should be adjusted or possibility that the patient should be admitted to a hospital for inpatient treatment.

Evaluating circulating levels of IL-33 in a subject typically includes obtaining a biological sample, e.g., serum or blood, from the subject. Levels of IL-33 in the sample can be determined by measuring levels of polypeptide, either the pre-IL33, proIL-33 or IL-33 peptide, in the sample, using methods known in the art and/or described herein, e.g., immunoassys such as enzyme-linked immunosorbent assays (ELISA). Alternatively, levels of IL-33 mRNA can be measured, again using methods known in the art and/or described herein, e.g., by quantitative PCR or Northern blotting analysis.

For example, a method as described herein, e.g., for diagnosis or prognosis of cardiac disease, can include contacting a sample from a subject, e.g., a sample including blood, serum, plasma, urine, or body tissue from the subject, with a binding composition (e.g., an antibody or oligonucleotide probe) that specifically binds to a polypeptide or nucleic acid of IL-33. The methods can also include contacting a sample from a control subject, normal subject, or normal tissue or fluid from the test subject, with the binding composition, e.g., to provide a reference or control. Moreover the method can additionally include comparing the specific binding of the composition to the test subject with the specific binding of the composition to the normal subject, control subject, or normal tissue or fluid from the test subject. Expression or activity of IL-33 in a test sample or test subject can also be compared with that in a control sample or control subject. A control sample can include, e.g., a sample from a non-affected subject, or it subject who has a cardiac disease of known seventy. Expression or activity from a control subject or control sample can be provided as a predetermined value, e.g., acquired from a statistically appropriate group of control subjects.

An antibody that "binds specifically to" an antigen, binds preferentially to the antigen in a sample containing other proteins. The term "antibody" as used herein refers to an immunoglobulin molecule or immunologically active portion thereof, i.e., an antigen-binding portion. Examples of immununologically active portions of immunoglobulin molecules include F(ab) and F(ab')₂ fragments which can be generated by treating the antibody with an enzyme such as pepsin. The antibody can be polyclonal, monoclonal, recombinant, e.g., a chimeric or humanized, fully human, non-human, e.g., murine, monospecific, or single chain antibody. In some embodiments it has effector function and can fix complement.

An "oligonucleotide probe" (also referred to simply as a "probe") is a nucleic acid that is at least 10, and less than 200 (typically less than about 100 or 50) base pairs in length. A probe that "binds specifically to" a target nucleic acid hybridizes to the target under high stringency conditions. As used herein, the term "hybridizes under high stringency conditions" describes conditions for hybridization and washing. As used herein, high stringency conditions are 0.5M sodium phosphate, 7% SDS at 65°C, followed by one or more washes at 0.2X SSC, 1% SDS at 65°C. Methods for performing nucleic acid hybridization assays are known to those skilled in the art and can be found in Current Protocols in Molcular Biology, John Wiley & Sons, N.Y. (1989), 6.3.1-6.3.6.

Detection can be facilitated by coupling (e.g., physically linking) the antibody or probe to a detectable substance (e.g., antibody labeling). Examples of detectable substances include various enzymes, prosthetic groups, fluorescent materials, luminescent materials, bioluminescent materials, and radioactive materials. Examples of suitable enzymes include horseradish peroxidase, alkaline phophatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride, quantum dots, or phycoerythrin; an example of a luminescent material includes luminol; examples of bioluminescent materials include luciferase, luciferin, and aequerin, and examples of suitble radioactive material include ¹²⁵I, ¹³¹I, ³⁵S or ³H.

Diagnostic assays can be used with biological matrices such as live cells, cell extracts, cell lysates, fixed cells, cell cultures, bodily fluids, or forensic samples. Conjugated antibodies useful for diagnostic or kit purposes, include antibodies coupled to dyes, isotopes, enzymes, and metals, see, e.g., Le Doussal et al., New Engl. J. Med. 146:169-175 (1991); Gibellini et al., J. Immunol. 160:3891-3898 (1998); Hsing and Bishop, Now Engl. J. Med. 162:2804-2811 (1999); and Everts et al., New Engl. J. Med. 168:883-889 (2002). Various assay formats exist, such as radioimmunosssays (RIA), ELISA, and lab on a chip (U.S. Pat. Nos. 6,176,962 and 6,517,234).

Known techniques in biochemistry and molecular biology can be used in the methods described herein (see, e.g., Maniatis et al., Molecular Cloning, A Laboratory Manual, Cold Spring Harbor Laboratory Press, Cold Spring Harbor, N.Y. (1982); Sambrook and Russell, Molecular Cloning, 3rd ed., Cold Spring Harbor Laboratory Press, Cold Spring Harbor, N.Y. (2001); Wu, Recombinant DNA, Vol. 217, Academic Press, San Diego, Calif (1993); and Ausbel et al., Current Photocols in Molecular Biology, Vols. 1-4, John Wiley and Sons, Inc. New York, N.Y. (2001)).

Once a level of IL-33 has been determined, the level is compared to a reference level. In some embodiments, e.g., where the level of IL-33 is determined using an ELISA, the reference level will represent a threshold level, above which the subject can be diagnosed with cardiac disease. The reference level chosen may depend on the methodology used to measure the levels of IL-33.

In some embodiments, the level of IL-33 is used to determine the level of severity of cardiac disease in a subject. The level of IL-33 typically increases with the severity of the cardiac disease, therefore, higher levels indicate more severe disease. Thus, the reference levels can represent levels of IL-33 in subjects with cardiac disease of known severity.

In some embodiments, the reference level is a range of levels.

In some embodiments, both levels of Interleukin 1 Receptor-Like 1 (IL1RL1) and IL-33 are determined, and the information from the comparison of both biomarkers with their respective reference levels provides cumulative information regarding the presence of cardiac disease in the subject, and/or the presence of a severe disease in the subject. In some embodiments, the ratio of ILIRL1 to IL-33 may be determined, and the ratio compared to a reference ratio that represents a threshold ratio above which the subject has cardiac disease.

Also described herein are kits that include a reagent for the detection of one or more of the IL-33 polypeptide(s) or nucleic acid, e.g., an anti-IL-33 antibody (i.e., an antibody that binds specifically to IL-33), or a nucleic acid probe complementary to all or part of the IL-33 nucleic acid, and instructions for use.

### Interleukin-33 (IL-33)

IL-33 was recently identified as the ligand for IL1RL1, and the presence of increased levels of IL-33 in various inflammatory disorders has been described (see Schmitz et al., Immunity 23(5):479-90 (2005); U.S. Pat. Pub. No. 2005/0203046).

IL-33 protein is expressed as an inactive molecule, pre-IL-33, that is activated after cleavage by Caspase I resulting in the active IL-33 peptide as well as the cleavage peptide product, pro-IL-33. Therefore, the methods described herein can includes measuring, one, two, or all three of mature IL-33, pro-IL-13, and/or pro-IL-33, all of which are included in the term "IL-33" as used herein.

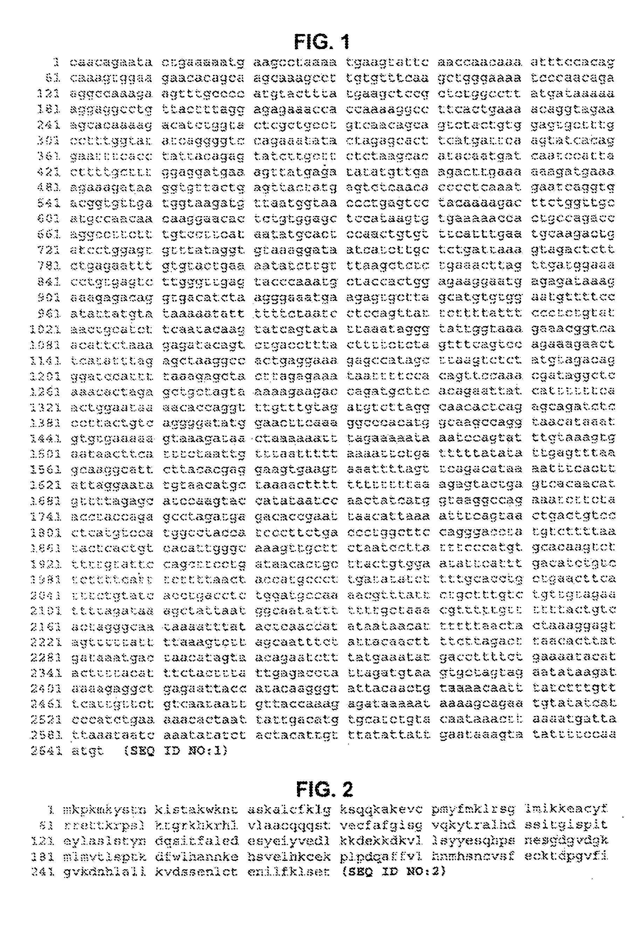

The nucleic acid sequence of human IL-33 can be found at GenBank Acc. No. NM_033439.2 (Figure 1, SEQ ID NO:1), and the polypeptide sequence is at GenBank Acc. No. NP_254274 (Figure 2, SEQ ID NO:2). Additional information is available in the public databases at GeneID: 90865, MIM ID # *608678, and UniGene No. Hs.348390. IL-33 is also known as Chromosome 9 Open Reading Frame 26 (C9ORP26); Nuclear Factor from High Endothelial Venules (NFHEV); and Interleukin 33. See also Baekkevold et al., Am. J. Path. 163: 69-79 (2003).

Methods for measuring levels of IL-33 polypeptide and nucleic acid are known in the art, see, e.g., Schmitz et al., Immunity 23(5):479-90 (2005); U.S. Pat. Pub. No. 2005/0203046,

### Interleukin 1 Receptor-like 1 (IL1RL1)

In the methods described herein, IL1 RL1 can be measured in addition to IL-33. The ratio of IL1 RL1 to IL-33 can also be determined. The IL1 RL1 gene is a member of the interleukin-1 receptor family, whose protein product exists both as a trans-membrane from, as well as a soluble receptor that is detectable in serum (Kieser et al., FEBS Lett. 372(2-3): 189-93 (1995); Kumar et al., J. Biol. Chem. 270(46):27905-13 (1993); Yanagisawa et al., FEBS Lett. 302(1):51-3 (1992); Kuroiwa et al., Hybridoma 19(2):151-9 (2000)). ST2 was recently described to be markedly up-regulated in an experimental model of heart failure (Weinberg et al., Circulation 106(23):2961-6 (2002)), and preliminary results suggest that ST2 concentration may be elevated in those with chronic severe HF (Weinherg et al., Circulation 107(5):721-6 (2003)) as well as in those with acute myocardial infarction (MI) (Shimpo et al., Circulation 109(18):2186-90 (2004)).

The transmembrane form of IL1RL1 is thought to play a role in modulating responses of T helper type 2 cells (Lohning et al., Proc. Natl. Acad. Sei. U. S. A. 95(12):6930-5 (1998); Schmitz et al., Immunity 23(5):479-90 (2005)), and may play a role in development of tolerance in states of seven or chronic inflammation (Brint et al., Nat, Immunol. 5(4):373-9 (2004)), while the soluble form of IL1RL1 is up-regulated in growth stimulated fibroblasts (Yanagisawa et al., 1992, supra). Experimental data suggest that the IL1RL1 gene is markedly up-regulated in states of myocyte stretch (Weinberg et al., 2002, supra) in a manner analogous to the induction of the BNP gene (Bruneau et al., Cardiovasc. Res. 28(10): 1519-25 (1994)).

Tominaga, FEBS Lett. 258:301-304 (1989), isolated murine genes that were specifically expressed by growth stimulation in BALB/c-3T3 cells; they termed one of these genes St2 (for Growth Stimulation-Expressed Gene 2). The St2 gene encodes two protein products: ST2, which is a soluble secreted form; and ST2L, a transmembrane receptor form that is very similar to the interleukin- 1 receptors. The HUGO Nomenclature Committee designated the human homolog, the cloning of which was described in Torminaga et al., Biochim. Biophys. Acta 1171:215-218 (1992), as Interleukin 1 Receptor-Like 1 (IL1RL1). The two terms are used interchangeably herein.

The mRNA sequence of the shorter, soluble isoform of human ST2 can be found at GenBank Acc, No. NM_003856.2, and the polypeptide sequence is at GenBank Acc. No. NP_003847.2; the mRNA sequence for the longer form of human ST2 is at GenBank Acc. No. NM_016232.4; the polypeptide sequence is at GenBank Acc. No. NP_057316.3. Additional information is available in the public databases at GeneID: 9173, MIM ID # 601203, and UniGene No. Hs.66. In general, in the methods described herein, the soluble form of ST2 polypeptide is measured.

Methods for detecting and measuring ST2 are known in the art, e.g., as described in U.S. Pat. Pub. Nos. 2003/0124624, 2004/0048286 and 2005/0130136. Kits for measuring ST2 polypeptide are also commercially available, e.g., the ST2 ELISA Kit manufactured by Medical & Biological Laboratories Co., Ltd. (MBL International Corp., Woburn, MA), no. 7638. In addition, devices for measuring ST2 and other biomarkers are described in U.S. Pat. Pub. No. 2005/0250156.

In some embodiment, the methods include determining the identity of the nucleotide sequence at RefSNP ID: rs1041973.

### Other Biomarkers

The methods described herein can also include measuring levels of other biomarkers in addition to IL-33. Suitable biomarkers include NT-proBNP, BNP, NT-proANP, and ANP troponio, GK-MB, Myo, IMA, IL-6, CRP, creatinine; D-dimers, and/or BUN,. Methods for measuring these biomarkers are known in the art, see, e.g., U.S. Pat. Pub. Nos. 2004/0048286 and 2005/0130136 to Lee et al.; Dhalla et al., Mol. Cell Biochem. 87:85-92 (1989); Moe et al., Am: Heart J. 139:587-95 (2000).

In these embodiments, levels of IL-33 and one or more additional biomarkers are determined, and the information from the comparison of the biomarkers with their respective reference levels provides additional information regarding the presence of cardiovascular disease in the subject, and/or the level of severity of the disease in the subject.

### Cardiovascular Disease

The methods described herein are useful in the diagnosis and prognosis of subjects with cardiovascular disease, e.g., MI, ACS, CAD, HF and/or stroke. In subjects with cardiovascular disease, a diagnosis is often made, and the extent of any cardiac tissue damage determined, using one or more of the following methods: electrocardiogram (ECG) - single or repeated over several hours; echocardiography; coronary angiography; nuclear ventriculography (e.g., radionuclide ventriculography (RNV) or multiple gate acquisition scan (MUGA)).

In addition, some biomarkers have proven to be by-products of heart damage or in conjunction with coronary artery disease (CAD), and therefore-useful for diagnosis of cardiac disease. These biomarkers include Troponin I (TnT) and troponin T (TnT); creatine phosphokinase (CPK) and CPK-MB; ischemia modified albumin (TMA) and serum myoglobin. The present invention provides additional methods of diagnosing cardiovascular disease, and prognostic methods for determining the severity of disease in a subject. Thus, the methods described herein can include determining IL-33 levels as part of a diagnostic effort, e.g., to determine whether a subject has cardiovascular disease and/orcan include determining IL-33 levels in a subject for whom a diagnosis of cardiovascular disease has already been made, e.g., to determine the severity of the disease.

Risk factors for cardiovascular disease include smoking, hypertension, high fat diet, poor blood cholesterol levels, especially high LDL ("bad") cholesterol and low HDL ("good") cholesterol, diabetes, male gender, age, heredity, and being overweight/obese. Biomarkers for increased risk include elevated homocysteine, C-reactive protein, and fibrinogen levels.

### Myocardial Infarction (M1)

A myocardial infarction (MI) occurs when an area of heart muscle becomes necrotic (dies) or is permanently damaged because of an madequate supply of oxygen to that area (ischemia). Most heart attacks are caused by a clot that blocks one of the coronary aneries, Clots usually form in a coronary artery that has been previously narrowed from changes related to atherosclerosis; atherosclerotic plaque (buildup) inside the arterial wall sometimes cracks, and this triggers the formation of a clot, also called a thrombus.

A clot in the coronary artery interrupts the flow of blood and oxygen to the heart muscic, resulting in ischemia of the tissue leading to the death of heart cells in that area (necrosis). The damaged heart muscle loses its ability to contract, and the remaining heart muscle needs to compensate for that weakened area

### Acute Coronary Syndrome (ACS)

ACS is a term that is used to cover any group of clinical symptoms associated with acute myocardial ischemia. Patients with ACS include those whose clinical presentations the following range of diagnoses: unstable angina, non- ST-elevation myocardial infarction (NSTEMI), and ST-elevation myocardial infarction (STEMI). Myocardial ischemia is most often due to atherosclerotic piques, as described above for MI.

### Heart Failure (HF)

Heart failure is a pathophysiologic state in which the heart, via an abnormality of cardiac function (detectable or not), fails to pump blood at a rate commensurate with the requirements of the metabolizing tissues, and/or pumps only from an elevated diastolic filling pressure.

Heart failure may be causal by myocardial failure but may also occur in the preserve of near-normal cardiac function under conditions of high demand, e.g., stress, Inadequate adaptation of the cardiac myocytes to increased wall stress to maintain adequate cardiac output following myocardial injury, is generally the meting event in CHF. HF may have an acute onset or may occur over several months to years, due to a primary disturbance in myocardial contractility or an excesssive hemodynamic burden placed on the ventricle, or both.

### Special Populations

Certain populations of may benefit particularly from the methods described herein. These subjects include people for whom BNP or NT-proBNP is less useful, such as in those with impaired renal function (Anwaruddin et al., J. Am. Coll. Cardiol. 47(1):91-7 (2006); McCullough et al., Am. J. Kidney Dis. 41 (3):571-9 (2003)), or in those who are overweight (Body Mass Index (BMI) of 25-29) or obese (BMI ≥ 30) (Krauser et al., Am. Heart J. 149(4):744-50 (2005); McCord et at, Arch. Intern. Med. 164(20):2247-52 (2004)). It is known and accepted in the field that patients with a high BMI usually have levels of natriuretic peptide that are lower than expected relative to a normal body mass patient for the same level of disease; the exact mechanism for this phenomerion is not known. IL-33 levels may not be affected by renal failure or high BMI. The methods described herein can include determining a subject's BMI, and if the subject is overweight or obese, selecting the patient for determination of IL-33 levels, as described herein.

### EXAMPLES

The invention is further described in the following examples, which do not limit the scope of the invention described in the claims.

### Example 1: Detection and Measurement of IL-33 in the Serum

A blood sample is collected from a subject, and serum is prepared from the sample using standard methods. A labeled monoclonal antibody to IL-33 (e.g., as described U.S. Pat. App. Pub. No. 2005/0203046, incorporated herein by reference in its entirety) in is added to the sample and incubated for a sufficient amount of time for binding to occur. The antibody/IL-33 complexes are then detected using standard methods, and the amount of IL-33 present is quantified.

### OTHER EMBODIMENTS

It is to be understood that while the invention has been described in with the detailed description thereof, the foregoing description is intended to illustrate and not limit the scope of the invention, which is defined by the scope of the appended claims. Other aspects, advantages, and modifications are within the scope of the following claims.

## Claims

1. An in vitro method of aiding in the diagnosis of a cardiovascular disease in a subject, the method comprising determining a level of IL-33 in a sample comprising blood, serum or plasma from a subject, wherein the level of IL-33 in the sample is indicative of whether the subject has a cardiovascular disease and further comprising comparing the level of IL-33 in the sample to a reference level, wherein the reference level represents a level in a subject who has or does not have cardiovascular disease.

2. An in vitro method of aiding in determining the severity of a cardiovascular disease in a subject, the method comprising determining a level of IL-33 in a sample comprising blood, serum or plasma from a subject, wherein the level of IL-33 in the sample is indicative of the severity of the cardiovascular disease in the subject and further comprising comparing the level of IL-33 in the sample to a reference level, wherein the reference level represents a level in a subject with a preselected severity of cardiovascular disease.

3. The method of claim 1 or 2, wherein determining a level of IL-33 in the sample comprises determining a level of one, two, or all three of mature IL-33, pre-IL-33, and pro-IL-33

4. The method of claim 1 or 2, wherein determining a level of IL-33 in the sample comprises contacting a binding composition to the sample, wherein the binding composition specifically binds to IL-33, and measuring or determining the specific binding of the binding composition to the sample.

5. The method of claim 4, wherein the binding composition comprises an antibody that binds specifically to IL-33 polypeptide.

6. The method of claim 5, wherein the binding composition comprises an oligonucleotide probe that binds specifically to IL-33 polynucleotide.

7. The method of claim 1 or 2, wherein the cardiovascular disease is acute coronary syndrome (ACS), myocardial infarction, heart failure, angina, cardiac hypertrophy, arteriosclerosis, myocarditis, pericarditis, endocarditis, stroke and/or pulmonary embolism.

8. The method of claim 1 or 2, further comprising determining a level in the sample of one or more other biomarkers.

9. The method of claim 8, wherein the other biomarkers are selected from the group casting of ST2, NT-proBNP, BNP, NT-proANP, and ANP, troponin, IMA, IL-6, CRP, creatinine, D-dimers, BUN, liver function enzymes, albumin. and bacterial endotoxin.

10. A method of monitoring the condition of a subject previously diagnosed with a cardiovascular condition, the method comprising:
determining a first level of IL-33 in a sample comprising blood, serum or plasma from a subject;
determining at least one subsequent level of IL-33 in a sample comprising blood, serum or plasma from the subject; and
comparing the level of IL-33 in the subsequent sample to the level of IL-33 in the first sample, wherein a difference in the IL-33 level in the subsequent sample relative to the level in the first sample is correlated with a change in the patient's condition.

11. The method of claim 10, wherein the subject is undergoing treatment on an outpatient basis.

12. The method of claim 10, wherein an increase in IL-33 in the subsequent sample relative to the level m the first sample indicates that the patient's condition is deteriorating.

13. The method of claim 12, wherein an increase in IL-33 in the subsequent sample relative to the level in the first sample indicates that the current treatment should be adjusted.

14. The method of claim 12, wherein an increase in IL-33 in the subsequent sample relative to the level in the first sample indicates that the patient should be admitted to a hospital for inpatient treatment.

## Patentansprüche

1. In vitro-Verfahren zum Unterstützen des Diagnostizierens einer kardiovaskulären Erkrankung bei einem Subjekt, wobei das Verfahren das Bestimmen eines IL-33-Spiegels in einer Probe, die Blut, Serum oder Plasma von einem Subjekt umfasst, wobei der IL-33-Spiegel in der Probe dafür indikativ ist, ob das Subjekt an einer kardiovaskulären Krankheit leidet, und des Weiteren das Vergleichen des IL-33-Spiegels in der Probe mit einem Bezugsspiegel umfasst, wobei der Bezugsspiegel einen Spiegel bei einem Subjet darstellt, das an einer kardiovaskulären Krankheit leidet oder nicht.

2. In vitro-Verfahren zum Unterstützen des Bestimmens der Schwere einer kardiovaskulären Erkrankung bei einem Subjekt, wobei das Verfahren das Bestimmen eines IL-33-Spiegels in einer Probe, die Blut, Serum oder Plasma von einem Subjekt umfasst, wobei der IL-33-Spiegel in der Probe für die Schwere der kardiovaskulären Krankheit bei dem Subjekt indikativ ist und des Weiteren das Vergleichen des IL-33-Spiegels in der Probe mit einem Bezugsspiegel umfasst, wobei der Bezugsspiegel einen Spiegel bei einem Subjekt darstellt, das an einer vorher gewählten Schwere einer kardiovaskulären Krankheit leidet.

3. Verfahren nach Anspruch 1 oder 2, wobei das Bestimmen eines IL-33-Spiegels in der Probe das Bestimmen eines Spiegels von einem, zwei oder allen drei von reifem IL-33, Prä-IL-33 und Pro-IL-33 umfasst.

4. Verfahren nach Anspruch 1 oder 2, wobei das Bestimmen eines IL-33-Spiegels in der Probe das Kontaktieren einer Bindungszusammensetzung mit der Probe, wobei die Bindungszusammensetzung sich spezifisch an IL-33 bindet, und das Messen oder Bestimmen der spezifischen Bindung der Bindungszusammensetzung an die Probe umfasst.

5. Verfahren nach Anspruch 4, wobei die Bindungszusammensetzung einen Antikörper umfasst, der sich spezifisch an IL-33-Polypeptid bindet.

6. Verfahren nach Anspruch 5, wobei die Bindungszusammensetzung eine Oligonucleotidsonde umfasst, die sich spezifisch an IL-33-Polynucleotid bindet.

7. Verfahren nach Anspruch 1 oder 2, wobei die kardiovaskuläre Krankheit das akute Koronarsyndrom (ACS), Herzinfarkt, Herzversagen, Angina, Herzhypertrophie, Arteriosklerose, Myokarditis, Perikarditis, Endokarditis, Schlaganfall und/oder Lungenembolie ist.

8. Verfahren nach Anspruch 1 oder 2, des Weiteren das Bestimmen eines Spiegels in der Probe eines oder mehrerer anderer Biomarker umfassend.

9. Verfahren nach Anspruch 8, wobei die anderen Biomarker aus der Gruppe ausgewählt sind bestehend aus ST2, NT-proBNP, BNP, NT-proANP und ANP, Troponin, IMA, IL-6, CRP, Creatinin, D-Dimeren, BUN, Leberfunktionsenzymen, Albumin und Bakterienendotoxin.

10. Verfahren zum Überwachen des Zustands eines Subjekts, das zuvor als an einer kardiovaskulären Beschwerde leidend diagnostiziert worden ist, wobei das Verfahren folgendes umfasst:
das Bestimmen eines ersten IL-33-Spiegels in einer Probe, die Blut, Serum oder Plasma von einem Subjekt umfasst;
das Bestimmen mindestens eines darauffolgenden IL-33-Spiegels in einer Probe, die Blut, Serum oder Plasma von einem Subjekt umfasst; und
das Vergleichen des IL-33-Spiegels in der darauffolgenden Probe mit dem IL-33-Spiegel in der ersten Probe, wobei ein Unterschied des IL-33-Spiegels in der darauffolgenden Probe mit Bezug auf den Spiegel in der ersten Probe mit einer Änderung des Zustands der Patienten korreliert.

11. Verfahren nach Anspruch 10, wobei das Subjekt eine Behandlung auf ambulanter Basis erfährt.

12. Verfahren nach Anspruch 10, wobei eine Erhöhung von IL-33 in der darauffolgenden Probe mit Bezug auf den Spiegel in der ersten Probe anzeigt, dass der Zustand des Patienten sich verschlechtert.

13. Verfahren nach Anspruch 12, wobei eine Erhöhung von IL-33 in der darauffolgenden Probe mit Bezug auf den Spiegel in der ersten Probe anzeigt, dass die gegenwärtige Behandlung angepasst werden sollte.

14. Verfahren nach Anspruch 12, wobei eine Erhöhung von IL-33 in der darauffolgenden Probe mit Bezug auf den Spiegel in der ersten Probe anzeigt, dass der Patient zur stationären Behandlung in ein Krankenhaus aufgenommen werden sollte.

## Revendications

1. Procédé *in vitro* pour faciliter le diagnostic d'une maladie cardio-vasculaire chez un sujet, le procédé comprenant la détermination d'un taux d'IL-33 dans un échantillon comprenant du sang, du sérum ou du plasma d'un sujet, dans lequel le taux d'IL-33 dans l'échantillon indique si le sujet a une maladie cardio-vasculaire, et comprenant en outre la comparaison du taux d'IL-33 dans l'échantillon avec un taux de référence, dans lequel le taux de référence représente un taux chez un sujet qui a ou n'a pas une maladie cardio-vasculaire.

2. Procédé *in vitro* pour faciliter la détermination de la sévérité d'une maladie cardio-vasculaire chez un sujet, le procédé comprenant la détermination d'un taux d'IL-33 dans un échantillon comprenant du sang, du sérum ou du plasma d'un sujet, dans lequel le taux d'IL-33 dans l'échantillon est indicatif de la sévérité de la maladie cardio-vasculaire chez le sujet, et comprenant en outre la comparaison du taux d'IL-33 dans l'échantillon avec un taux de référence, dans lequel le taux de référence représente un taux chez un sujet ayant une sévérité présélectionnée de maladie cardio-vasculaire.

3. Procédé selon la revendication 1 ou 2, dans lequel la détermination d'un taux d'IL-33 dans l'échantillon comprend la détermination d'un taux d'une, de deux ou de toutes les trois parmi IL-33 mature, pré-IL-33 et pro-IL-33.

4. Procédé selon la revendication 1 ou 2, dans lequel la détermination d'un taux d'IL-33 dans l'échantillon comprend la mise en contact d'une composition de liaison avec l'échantillon, dans lequel la composition de liaison se lie spécifiquement à IL-33, et la mesure ou la détermination de la liaison spécifique de la composition de liaison à l'échantillon.

5. Procédé selon la revendication 4, dans lequel la composition de liaison comprend un anticorps qui se lie spécifiquement au polypeptide IL-33.

6. Procédé selon la revendication 5, dans lequel la composition de liaison comprend une sonde oligonucléotidique qui se lie spécifiquement au polynucléotide IL-33.

7. Procédé selon la revendication 1 ou 2, dans lequel la maladie cardio-vasculaire est un syndrome coronaire aigu (SCA), un infarctus du myocarde, une défaillance cardiaque, une angine de poitrine, une hypertrophie cardiaque, une artériosclérose, une myocardite, une péricardite, une endocardite, une attaque et/ou une embolie pulmonaire.

8. Procédé selon la revendication 1 ou 2, comprenant en outre la détermination d'un taux d'un ou plusieurs autres biomarqueurs dans l'échantillon.

9. Procédé selon la revendication 8, dans lequel les autres biomarqueurs sont choisis dans le groupe constitué par ST2, NT-proBNP, BNP, NT-proANP et ANP, troponine, IMA, IL-6, CRP, créatinine, D-dimères, BUN, enzymes de la fonction hépatique, albumine et endotoxine bactérienne.

10. Procédé pour suivre l'affection d'un patient préalablement diagnostiqué avec une affection cardio-vasculaire, le procédé comprenant:
la détermination d'un premier taux d'IL-33 dans un échantillon comprenant du sang, du sérum ou du plasma d'un sujet;
la détermination d'au moins un taux subséquent d'IL-33 dans un échantillon comprenant du sang, du sérum ou du plasma du sujet; et
la comparaison du taux d'IL-33 dans l'échantillon subséquent avec le taux d'IL-33 dans le premier échantillon,
dans lequel une différence entre le taux d'IL-33 dans l'échantillon subséquent et le taux dans le premier échantillon est corrélée avec un changement de l'affection du patient.

11. Procédé selon la revendication 10, dans lequel le sujet subit un traitement de façon ambulatoire.

12. Procédé selon la revendication 10, dans lequel une augmentation d'IL-33 dans l'échantillon subséquent par rapport au taux dans le premier échantillon indique que l'affection du patient se détériore.

13. Procédé selon la revendication 12, dans lequel une augmentation d'IL-33 dans l'échantillon subséquent par rapport au taux dans le premier échantillon indique que le traitement en cours devrait être ajusté.

14. Procédé selon la revendication 12, dans lequel une augmentation d'IL-33 dans l'échantillon subséquent par rapport au taux dans le premier échantillon indique que le patient devrait être admis dans un hôpital pour un traitement avec hospitalisation.
